# EUROPEAN PATENT APPLICATION

(11) **EP 2 628 505 A1**
(43) Date of publication of application: **21.08.2013**
(21) Application number: 11832177.7
(22) Date of filing: 13.10.2011
(51) Int. Cl.: A61N 2/12

(54) **MEDICAL DEVICE**

(30) Priority: 14.10.2010 ES 201031019 U
(71) Applicant: Fradera Pellicer, Carlos, L'Aldosa, La Massana (AD)
(72) Inventor: Fradera Pellicer, Carlos, L'Aldosa, La Massana (AD)
(74) Representative: Curell Suñol S.L.P.
(86) International application number: PCT/ES2011/070707
(87) International publication number: WO 2012/049348

(57) **Abstract**

Medical equipment comprising a device generating a magnetic field intended to be applied to a patient and a support structure (2) for said patient. A head (6) generating the magnetic field is associated to said support structure (2), and can be moved gradually and repeatedly in a guided manner mechanically and/or manually, in a position at a distance from the patient, describing a trajectory defined by a supporting guide (5) attached to said support structure (2). The trajectory described by head (6) runs in the transverse and/or longitudinal direction along the surface of the patient's body to be treated with the magnetic field.

## Description

### Field of the invention

This invention relates to medical equipment comprising a magnetic field generator intended to be used on a patient and a support structure for said patient.

### State of the art

A plurality of devices are known fitted with means for creating a magnetic field for therapeutic purposes, using either magnets or electromagnets.

In most cases, the means generating the magnetic field are manual apparatuses used by the health care staff who apply them to a certain point on the patient or move them over an area of the patient. Document ES2206025 discloses a device of this type that uses electromagnets, while documents MX2009000565, DE4132078, CA2526977, CA2282723, WO99/19022 and US5562706 disclose devices of this mentioned type that use permanent magnets.

In other cases, less frequent than in the above case, the means generating the magnetic field are located in an area of a stretcher, either under the patient or above him, and can rotate or oscillate around a vertical axis to project their magnetic emission over a limited area of the patient. Document ES2112777 discloses a device of this type, wherein the magnetic field is created by means of solenoids. Documents US4727857 and US5667469 disclose devices of this type wherein permanent magnets are used. Document DE10032814 discloses a device of this type wherein some magnets oscillate in swinging movement under a stretcher.

Finally, in other cases, the magnetic field generator is fixed to a point and it is the patient who, sitting or standing, places him in front of the generating device. Document DE 3221544 discloses a device of this type wherein a disc with permanent magnets is driven by a motor and it is located in a support foot.

In examining these background devices, it is revealed that the means generating the magnetic field are located at a point on the stretcher or the like, or they move along a fixed trajectory with respect to the stretcher or treatment table under the patient, and therefore the patient must be moved according to the surface of the patient's body where it is desired to apply the treatment.

### Disclosure of the invention

So that the patient does not have to be moved from one area to another on the surface of the treatment table or stretcher where he is sitting, the solution has been adopted where the head generating the magnetic field is moved in guided fashion above or under, in front of or behind the patient or around the patient, always facing an area to be treated using successive, repeated steps with the head.

According to the preceding solution the medical equipment that is the object of the invention has been developed, which comprises a magnetic field generator intended to be applied to a patient and a support structure (2, 14) for said patient, and it is characterised in that it comprises, associated to said support structure, a head generating a magnetic field, with said head being able to move, gradually and repeatedly in a guided manner and/or manually, in a position at a distance from the patient, describing a trajectory defined by a supporting guide attached to said support structure, with said trajectory running in the transversal and/or longitudinal direction along the surface of the body of the patient to be treated with said magnetic field.

In some preferred embodiments, said support structure is associated to a stretcher structure, preferably to a stretcher suitable for inverting the patient.

Preferably, said supporting guide, intended to allow the mechanical movement along it of said head generating the magnetic field, consists of a resistant element that can be positioned in front of any surface on the patient's body and which has a longitudinal constitution, with an arrangement belonging to the group comprising a curvilinear shape, a rectilinear shape and combinations thereof, with said resistant element being arranged so that it extends facing, on its face nearest the patient and irrespective of whether this is rectilinear or concave, a part of the surface of the patient's body that must be treated with the magnetic field.

Preferably, said head generating a magnetic field runs via said supporting guide so that the emitting face of the magnetic field is always orientated towards the surface of the patient's body to be treated.

Preferably, said head generating a magnetic field is provided with means for moving along the trajectory defined by said supporting guide, which belong to the group comprising the autonomous means and the external means, whether considered individually or in technically feasible combinations.

Preferably, said head generating a magnetic field comprises a support which includes magnets emitting magnetic fields with a defined polarity, belonging to the group comprising magnets with homogeneous polarity in power and alternate directions, magnets with homogeneous power polarity and in the same direction and magnets with heterogeneous polarities in power and/or direction, considered individually or in technically feasible combinations thereof.

Preferably, said support that includes the magnets of the head generating a magnetic field is assembled on a rotary shaft related to a driving force.

In some embodiments, said supporting guide is provided with means for installing it at chosen points on said support structure where the patient is located in the stretched out position, with said support structure being one from the group comprising a conventional stretcher and an inverting stretcher, considered individually or in technically feasible combinations thereof.

In other embodiments, said support structure in which said supporting guide is installed is a vertical support structure where the patient is located in the upright or seating position.

Preferably, said head generating a magnetic field is configured to project a magnetic emission with a value between 500 and 2800 gauss.

Preferably, the medical equipment according to the invention is configured so that said support that includes the magnets can rotate with a rotation speed between 0 and 1500 rpm.

Preferably, said magnets are from the group comprising neodymium magnets, ferrite magnets and electromagnets, considered individually or in technically feasible combinations thereof.

Preferably, said head generating a magnetic field is associated with means for rolling on said supporting guide, which are likely to be associated with controlled movement motor means along said supporting guide.

Preferably, said head generating a magnetic field is provided with a gripping handle for manual use which is free of rolling means, motors and the supporting guide.

Preferably, said head comprises a plurality of magnets arranged with their magnetic axis orientated so that it is perpendicular to the emitting face of said head, where part of said magnets have their north pole on said emitting face and the remaining magnets have their south pole on said emitting face.

In some preferred embodiments, said head has a plurality of pairs of magnets, preferably four magnets, arranged in a circle, where each of said magnets that has its north pole on said emitting face has adjacent to it two of said magnets that have their south pole on said emitting face.

Preferably, the medical equipment according to the invention comprises a plurality of said heads generating a magnetic field associated to said support structure.

In the equipment according to the invention, the head can include a magnetic configuration with very different shapes and can include permanent magnets, electromagnets or combinations of both. In this description and in the claims the term "magnet" or "magnets" is used to indicate any of the above possibilities.

### Brief description of the drawings

To facilitate the understanding of the expounded ideas, while also disclosing various construction details, an embodiment of this invention is described below with reference to the drawings accompanying this specification, which given their essentially illustrative purpose, must be interpreted as not limiting in any way the scope of legal protection sought. The drawings show as follows:
Fig. 1 represents a top perspective view of a inverting stretcher structure that includes the medical equipment that is the object of the invention, during the treatment of a patient.
Fig. 2 represents a side elevation view of the stretcher in the preceding figure in its inverted position, in which the treatment can be carried out once the medical equipment has been installed appropriately.
Fig. 3 represents a side elevation view of an embodiment of the head generating the magnetic field assembled in cantilever arrangement on a tubular supporting guide.
Fig. 4 represents a top perspective view of a possible embodiment of the medical equipment wherein the support structure consists of a vertical bar or column that supports, at the height required in each case, the supporting guide of the head generating the magnetic field. In the figure, the patient receives the treatment in the standing up position, but could also be in the sitting position.
Fig. 5 represents a side elevation view, partially sectioned, of a head generating the magnetic field, dismantled from the supporting guide in order to be used for an extraordinary treatment manually. The figure shows the arrangement of the magnets.
Fig. 6 represents a bottom plan view of the head generating the magnetic field in the preceding figure.

### Description of some embodiments of the invention

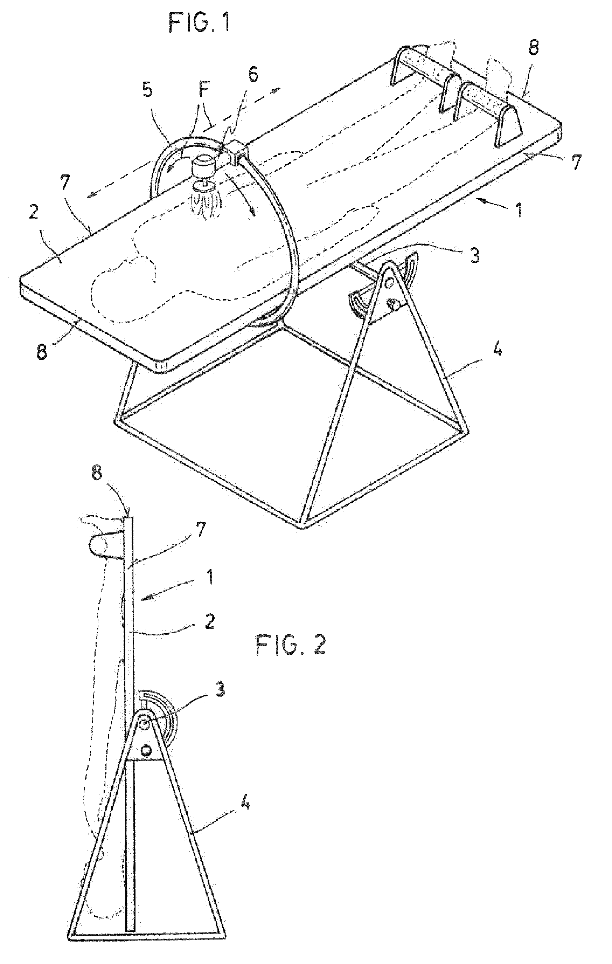

Figure 1 shows a support structure for a patient consisting of an inversion stretcher 1 made up of a support plate 2 that swings around a shaft 3 on a support 4. Support plate 2 has end stops for retaining the patient's feet so that said support plate 2 can be arranged vertically, with the patient's feet in the top position, as shown in Figure 2. In this position the patient's spine is advantageously stretched longitudinally.

In inverted stretcher 1 a supporting guide 5 is provided for moving a head 6 generating a magnetic field.

In Figure 1 supporting guide 5 is installed transverse to support plate 2 and attached to the sides thereof.

The invention contemplates that supporting guide 5 can be located transverse to support plate 2, at any point on sides 7 thereof, or longitudinally at any point of ends 8 thereof, so that any area of the surface of the patient's body can be reached, as indicated with the arrows in Figure 1.

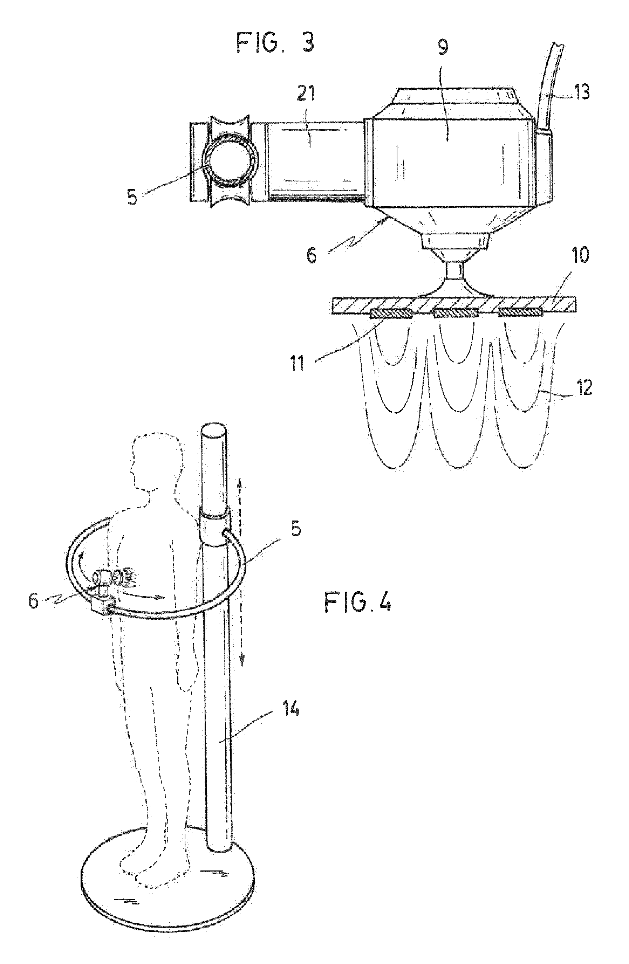

Figure 3 shows an embodiment of a head 6 generating a magnetic field assembled in a sliding manner on supporting guide 5, which has a tubular section. In the figure it be seen the housing 9 of a motor that determines the rotation at a controlled speed of a plate 10 containing magnets 11 that emit magnetic fields 12. Said figure also shows a electrical conductor 13 to feed said motor.

Figure 4 illustrates a possible embodiment of the medical equipment, wherein support structure 2 in Figures 1 and 2 has been replaced with a vertical bar or column 14 that supports, at the convenient height determined in each case for the specific treatment to be carried out, supporting guide 5 that supports head 6 generating the magnetic field. The patient receives the therapy in the standing up position, but could also be in the sitting position.

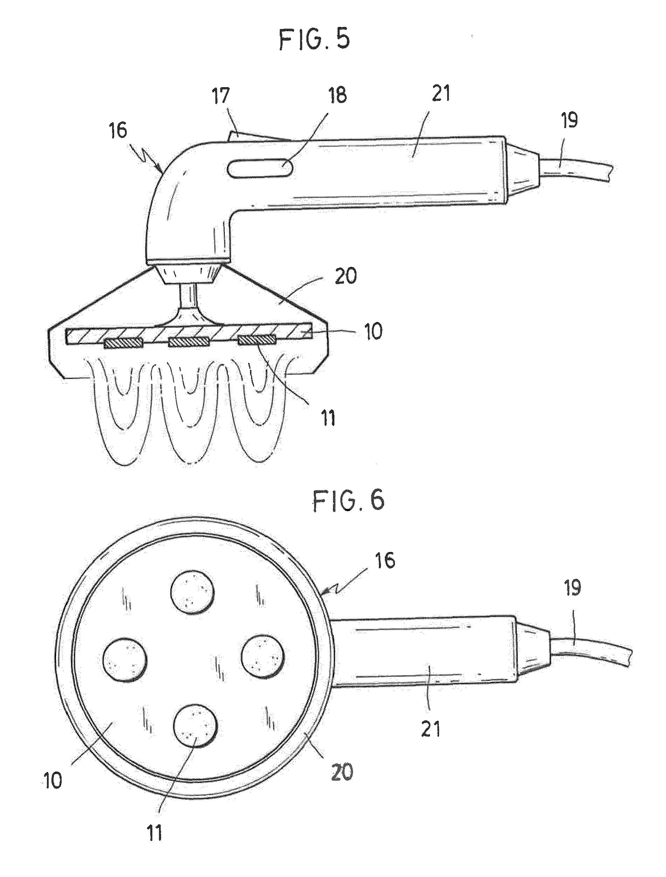

Figures 5 and 6 illustrate a head 16 generating the magnetic field that is different from head 6, because it consists of a head suitable for being used manually for an extraordinary treatment, without excluding the possibility of allowing that head 16 be incorporated into a supporting guide 5. Head 16 is provided with plate 10 carrying magnets 11, an action control 17, means 18 for its possible association 18 to supporting guide 5 and a gripping handle 21. Also observed in the figure is an electrical conductor 19 to feed the motor. Plate 10 together with magnets 11 is housed in a partial shell 20.

Heads 6 and 16 generating the magnetic field are capable of projecting individually a magnetic emission with a value between 500 and 2800 gauss.

Plate 10 that supports magnets 11 can rotate at a rotary speed between 0 and 1500 rpm.

Magnets 11 are one from the group comprising neodymium magnets, ferrite magnets and electromagnets, considered individually or in technically feasible combinations thereof.

Head 6 generating a magnetic field is associated with means for rolling on supporting guide 5, which are likely to be associated with motor means controlling movement thereon.

Head 16 generating a magnetic field is provided with a gripping handle 21 for its manual use, and therefore it does not have rolling or motor means for moving on a supporting guide 5. However, it is provided with association means 18 for being attached to a rolling device to roll on a supporting guide 5.

## Claims

1. Medical equipment comprising a magnetic field generator intended to be applied to a patient and a support structure (2, 14) for said patient, **characterised in that** it comprises, associated to said support structure (2, 14), a head (6, 16) generating a magnetic field, with said head (6, 16) being able to move, gradually and repeatedly in a guided manner and/or manually, in a position at a distance from the patient, describing a trajectory defined by a supporting guide (5) attached to said support structure (2, 14), with said trajectory running in the transversal and/or longitudinal direction along the surface of the body of the patient to be treated with said magnetic field.

2. Medical equipment according to claim 1, **characterized in that** said support structure (2) is associated to a stretcher structure (1), preferably a stretcher suitable for inverting the patient.

3. Medical equipment according to one of the claims 1 or 2, **characterized in that** said supporting guide (5), intended to allow the mechanical movement along it of said head (6, 16) generating a magnetic field, consists in a resistant element that can be positioned in front of any surface on the patient's body and which has a longitudinal constitution, with an arrangement belonging to the group comprising a curvilinear shape, a rectilinear shape and combinations thereof, with said resistant element being arranged so that it extends facing, on its face nearest the patient and irrespective of whether this is rectilinear or concave, a part of the surface on the patient's body that must be treated with the magnetic field.

4. Medical equipment according to any of the claims 1 to 3, **characterized in that** said head (6, 16) generating a magnetic field runs via said supporting guide (5) so that its face emitting the magnetic field is always orientated towards the surface of the patient's body to be treated.

5. Medical equipment according to any of the claims 1 to 4, **characterized in that** said head (6, 16) generating a magnetic field is provided with means for moving along the trajectory defined by said supporting guide (5), which belong to the group comprising autonomous means and external means, either considered individually or in technically feasible combinations thereof.

6. Medical equipment according to any of the claims 1 to 5, **characterized in that** said head (6, 16) generating a magnetic field comprises a support (10) that includes magnets (11) emitting magnetic fields with defined polarity, belonging to the group comprising magnets with homogeneous polarity in power and alternate directions, magnets with homogeneous power polarity and in the same direction and magnets with heterogeneous polarities in power and/or direction, considered individually or in technically feasible combinations thereof.

7. Medical equipment according to la claim 6, **characterized in that** said support (10) that includes magnets (11) of head (6, 16) generating a magnetic field is assembled on a rotary shaft related to a motor element.

8. Medical equipment according to any of the claims 1 a 7, **characterized in that** said supporting guide (5) is provided with means for installing it at chosen points on said support structure (2) where the patient is located in the stretched out position, with said support structure being one from the group comprising a conventional stretcher and an inverting stretcher, considered individually or in technically feasible combinations thereof.

9. Medical equipment according to any of the claims 1 to 7, **characterized in that** said support structure (14) wherein said supporting guide (5) is installed is a vertical support structure wherein the patient is located in the upright or sitting position.

10. Medical equipment according to any of the claims 1 to 9, **characterized in that** said head (6, 16) generating a magnetic field is configured to project a magnetic emission with a value between 500 and 2800 gauss.

11. Medical equipment according to claim 7, **characterized in that** it is configured so that said support (10) that includes magnets (11) can rotate at a rotation speed between 0 and 1500 rpm.

12. Medical equipment according to claim 6, **characterized in that** said magnets (11) are ones from the group comprising neodymium magnets, ferrite magnets and electromagnets, considered individually or in technically feasible combinations thereof.

13. Medical equipment according to any of the claims 1 to 12, **characterized in that** said head (6) generating a magnetic field is associated with means for rolling on said supporting guide (5), which are likely to be associated to motor means for a controlled movement along said supporting guide (5).

14. Medical equipment according to any of the claims 1 to 12, **characterized in that** said head (16) generating a magnetic field is provided with a gripping handle (21) for manual use which is free of rolling means, motors and the supporting guide.

15. Medical equipment according to any of the claims 1 to 14, **characterized in that** said head (6, 16) comprises a plurality of magnets (11) arranged with their magnetic axis orientated so that it is perpendicular to the emitting face of said head (6, 16), where part of said magnets (11) have their north pole on said emitting face and the remaining magnets have their south pole on said emitting face.

16. Medical equipment according to claim 15, **characterized in that** said head (6, 16) has a plurality of pairs of magnets (11), preferably four magnets (11), arranged in a circle, where each of said magnets (11) that has its north pole on said emitting face has adjacent to it two of said magnets (11) that have their south pole on said emitting face.

17. Medical equipment according to any of the claims 1 to 16, **characterized in that** it comprises a plurality of said head (6, 16) generating a magnetic field associated to said support structure (2, 14).
